Europäisches Patentamt

European Patent Office

Office européen des brevets

(11) Numéro de publication: **0 002 977**
**A1**

(12) **DEMANDE DE BREVET EUROPEEN**

(21) Numéro de dépôt: **78400234.7**

(22) Date de dépôt: **13.12.78**

(51) Int. Cl.²: **C 07 D 301/10, B 01 J 23/50,
B 01 J 35/10, C 07 D 303/04**

(30) Priorité: **22.12.77 FR 7738744**

(43) Date de publication de la demande: **11.07.79**
**Bulletin 79/14**

(84) Etats contractants désignés: **BE DE FR GB NL**

(71) Demandeur: **PRODUITS CHIMIQUES UGINE
KUHLMANN, Service Propriété Industrielle Tour
Manhattan, F-92087 PARIS LA DEFENSE 2
Cédex 21 (FR)**

(72) Inventeur: **Kervennal, Jacques, 134 rue Docteur
Locard, F-69005 Lyon (FR)**
Inventeur: **Cognion, Jean-Marie, M., 85 route de Charly,
F-69230 St Genis Laval (FR)**

(74) Mandataire: **Monceaux, Pierre et al, PRODUITS
CHIMIQUES UGINE KUHLMANN Service Propriété
Industrielle, Tour Manhattan Cédex 21 F-92087 Paris
La Defense (FR)**

(54) **Catalyseurs à base d'argent pour la production d'oxyde d'éthylène.**

㊼ Catalyseurs à base d'argent pour la synthèse en phase vapeur d'oxyde d'éthylène par réaction d'oxygène ou de mélanges gazeux en contenant sur de l'éthylène, caractérisés par l'emploi comme support d'un matériau poreux présentant une surface spécifique inférieure à 10 m₂/g, un volume total de porosité compris entre 0,1 et 0,6 cm³/g et une répartition bimodale de la porosité où les plus petits pores représentant 35 à 65% du volume des pores ont un diamètre moyen compris entre 1 et 5 μ, les gros pores ayant un diamètre moyen compris entre 60 et 200 μ.

ACTORUM AG

- 1 -

Catalyseurs à base d'argent pour la
production d'oxyde d'éthylène

La présente invention concerne des catalyseurs à base d'argent sur support employés dans l'époxydation en phase vapeur des oléfines et plus particulièrement pour la production, par cette technique, de l'oxyde d'éthylène à partir
d'éthylène et d'oxygène moléculaire.

De façon générale la production d'oxyde d'éthylène est effectuée en phase vapeur, dans des réacteurs tubulaires à lit
fixe, par réaction de l'oxygène et de l'éthylène sur des
phases catalytiques à base d'argent déposées sur des supports
réfractaires et inertes formés principalement d'alumine, de
silice-alumine, de magnésie, de pierre-ponce, de zircone,
d'argile, de céramique, de graphite naturel ou artificiel,
d'amiante, de zéolithe naturelle ou artificielle, ou de carbure de silicium. L'art antérieur préfère généralement les
solides de faibles surfaces spécifiques et tous les supports
revendiqués ont des surfaces inférieures à 10 m2/g. C'est
ainsi que l'on trouve cité, dans le brevet français
n° 2.253.747, toute une série de produits poreux : carbure
de silicium, zircone, silice, silice-alumine, alumine α de
surfaces comprises entre 0,04 et 10 m2/g. Des surfaces spécifiques voisines : 0,03 à 10 m2/g, sont également revendiquées dans les brevets français n° 2.117.183, 2.130.465,

0002977

- 2 -

2.167.728, 2.249.087 et 2.271.869 à l'aide de supports commercialisés tous à base d'alumine ou de silice - alumine. En
fait les surfaces spécifiques des supports employés sont
nettement plus faibles et des valeurs inférieures au m2/g
sont revendiquées dans les brevets français n° 2.243.193
(alumine, carbure de silicium, silice-alumine) et
n° 1.522.279 (silice-alumine). Ces surfaces spécifiques sont
déterminées dans la majorité des cas, par la méthode d'adsorption de l'azote, méthode dite B.E.T. décrite par
BRUNAUER, EMMET et TELLER dans "the journal of the american
chemical Society" vol. 60, page 309, 1938. La deuxième caractéristique importante de ces supports est la porosité.
Tous les auteurs sont d'accord pour admettre qu'une porosité,
pouvant atteindre 60 % en volume est favorable à l'activité
et à la sélectivité en oxyde d'éthylène des catalyseurs obtenus. Par contre une certaine dispersion existe dans les
revendications sur les dimensions des pores.

Elle est nettement mise en évidence par les chiffres suivants :
- 0,2 à 0,4 microns dans le brevet français n° 2.249.087.
- 1 à 15 microns dans les brevets français n° 2.253.747,
2.117.183 et dans le brevet belge n° 848.659 pour des supports alumine ou silice-alumine de faibles granulométries.
- 50 à 200 microns dans les brevets français n° 1.354.391 et
1.413.213 pour des supports alumine ou carbure de silicium
de granulométrie importante pouvant atteindre 9 mm.
- 10 à 300 microns dans les brevets français n° 2.243.193,
2.023.984 et 2.208.713 et le brevet anglais n° 1.133.484.
- 50 à 1.500 microns dans les brevets français n° 2.029.751,
2.059.124 et le brevet tchèque n° 130.654 pour des supports
à base d'alumine de granulométrie élevée, pouvant atteindre
9 mm.

L'importance de la relation entre le diamètre des pores et la granulométrie des catalyseurs de synthèse de l'oxyde d'éthylène a été signalée dans deux brevets : le brevet français n° 2.253.747 qui signale que l'augmentation de la granulométrie des supports silice-alumine doit être accompagnée d'une augmentation du diamètre moyen des pores et la demande de brevet français n° 77/21.118 de la demanderesse qui revendique, pour des supports graphites artificiels, l'effet bénéfique d'une augmentation du diamètre des macropores avec la granulométrie.

La demanderesse a découvert en plus et c'est l'objet de la présente invention, que l'époxydation catalytique de l'éthylène en lit fixe pouvait être effectuée avec de très bonnes sélectivités par l'emploi, comme supports des phases actives à base d'argent, de matériaux poreux de surface spécifique inférieure à 10 m2/g, de volume de porosité totale pouvant atteindre 60 % en volume, de granulométrie supérieure au mm et possédant une répartition bimodale de la porosité. Une telle répartition signifiant que la porosité du support est formée de deux groupes de pores différant par leur diamètre.

La demanderesse a ainsi trouvé que, pour des catalyseurs employés à lit fixe, de granulométrie comprise entre 1 et 15 mm, et plus particulièrement entre 3 et 10 mm, la coexistence de deux domaines de porosité formée de pores de diamètres différents était favorable à la sélectivité en oxyde d'éthylène. Le diamètre moyen des pores formant ces deux domaines de porosité se situe d'une part dans la zone de 1 à 5 microns et d'autre part dans la zone de 60 à 200 microns. Chacun de ces deux domaines de porosité représente de façon préférentielle au minimum 25 % et au maximum 75 % de la porosité totale.

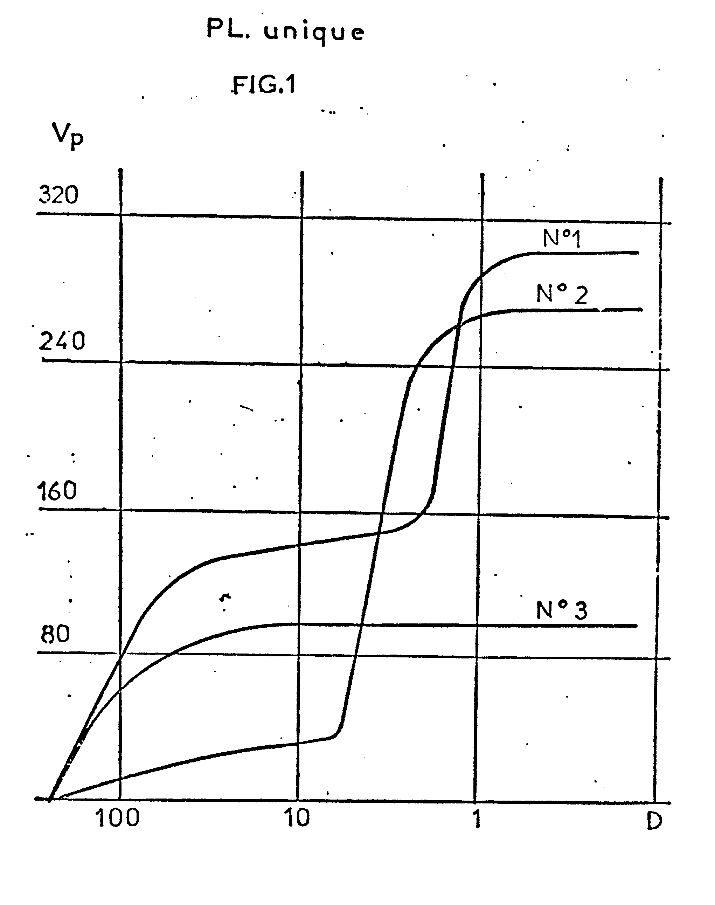

La supériorité des catalyseurs, objet de la présente invention, a été clairement mise en évidence par des essais comparatifs sur trois supports commercialisés par la firme NORTON sous les dénominations SA 5239, SA 5151 et SA 5205, et dont un seul le SA 5239 possède une répartition bimodale de la porosité et illustre de façon non limitative la présente invention. Les caractéristiques de ces supports sont rassemblées dans le tableau 1 et sur la figure 1 qui donne la variation des volumes de porosité, exprimée en cm3/g en fonction du diamètre des pores, exprimés en microns.

0002977

## TABLEAU n° 1

Vp : volume de porosité

$R_M$ : rayon moyen des pores

| SUPPORT | | SA 5239 | SA 5151 | SA 5205 |
|---|---|---|---|---|
| Forme granulométrique | | sphères 1/4 pouce (6,4 mm) | extrudées 1/4 x 1/4 pouce (6,4 x 6,4 mm) | sphères 1/4 pouce (6,4 mm) |
| $Al_2O_3$ | % | 86,4 | 99,3 | 86,1 |
| $SiO_2$ | % | 12 | 0,4 | 11,8 |
| CaO | % | 0,2 | 0,1 | 0,4 |
| $Na_2O$ | % | 0,4 | 0,1 | 0,4 |
| $K_2O$ | % | 0,2 | - | 0,6 |
| Surface spécifique BET (m2/g) | | 0,30 | 0,20 | 0,30 |
| POROSITE : | | | | |
| Vp total (cm3/g) | | 0,340 | 0,270 | 0,100 |
| Vp (R = 0,25 - 5 µ) | | 0,160 (47 %) | 0,240 (89 %) | 0 0 |
| Vp (R = 5 - 100 µ) | | 0,180 (53 %) | 0,030 (11 %) | 0,100 (100 %) |
| $R_M$ des pores (µ) | | 0,7 et 50 | 2 | 55 |
| Figure : | | Support n° 1 | Support n° 2 | Support n° 3 |

Ces mesures de porosité ont été effectuées par la méthode dite du porosimètre à mercure, préconisée par E. W. WASHBURN dans les Proceedings of the National Academy of Sciences of the U. S. A., vol. 7, page 115, 1927, et décrite par L. C. DRAKE dans Industrial and Engineering Chemistry, vol. 41, page 780, 1949 et dans Industrial and Engineering Chemistry Analytical Edition, vol. 17, page 782, 1945.

La nature des supports a également son importance. Dans le cadre de la présente invention, on peut employer des supports réfractaires, plus ou moins bons conducteurs comme l'alumine α, les silice-alumines, le carbure de silicium, la zircone ou le graphite. Ils peuvent se présenter sous forme d'anneaux, de pastilles, de cassons, de sphères ou de formes extrudées de propriétés mécaniques satisfaisantes, ne permettant en aucun cas la formation de poussières au cours de la préparation du catalyseur, de ses manipulations ou de son fonctionnement.

Les surfaces spécifiques inférieures à 10 m2/g sont avantageusement comprises entre 0,1 et 1 m2/g et les volumes de porosité totale qui peuvent atteindre 60 % en volume sont préférentiellement compris entre 20 et 50 %.

La préparation de ces nouveaux catalyseurs ne pose aucun problème et peut être réalisée par tout procédé classique. On peut en particulier opérer de façon connue de l'homme de l'art en deux étapes par imprégnation ou enrobage d'un composé de l'argent en solution ou suspension dans un solvant volatil, suivi d'un traitement permettant le passage sur le support au métal.

Les composés d'argent utilisés peuvent être soit des sels : nitrate, formiate, lactate, citrate, carbonate, oxalate, acétate, sulfate, propionate, maléate, malate, malonate, phtalate, picolinate, anthranilate, tartrate, glycolate, succinate, oxyde, hydroxyde, acétylure ou céténure, soit des complexes de sels d'argent avec les molécules azotées et/ou oxygénées comme l'ammoniac, l'acrylonitrile, la pyridine, l'éthanolamine, l'éthylène diamine, soit des complexes d'argent avec des molécules organiques telles que l'acide salicylique, le salicylaldéhyde, les β-dicétones et les β-cétoesters. Les principaux solvants ou liquides de suspension employés sont l'eau, l'acétone, les alcools légers, l'éther, la pyridine, l'éthylène glycol, le diéthylène glycol ou les solvants chlorés.

Toutes les techniques qui permettent le passage de ces composés au métal ou à l'oxyde peuvent être appliquées, par exemple, la précipitation, la décomposition thermique en atmosphère inerte, oxydante ou réductrice et la réduction chimique.

Les catalyseurs, objet de la présente invention, ont été préparés préférentiellement par un procédé comprenant les trois étapes suivantes :

a) imprégnation du support avec une solution du composé d'argent choisi. Cette imprégnation pouvant être effectuée soit par immersion du support dans la solution, soit par arrosage continu du support sous pression réduite et à une température permettant l'élimination immédiate du solvant.

b) séchage du support imprégné.

c) traitement thermique du produit obtenu de manière à libérer l'argent du composé déposé sur le support.

L'éventuelle addition des promoteurs habituels alcalins ou alcalino-terreux est bénéfique et peut se faire lors de l'imprégnation. Il est ainsi possible d'ajouter à ces catalyseurs, dans les teneurs habituelles de 0 à 2 % en poids, un ou plusieurs promoteurs classiques de l'argent décrits dans l'art antérieur et choisis par exemple parmi les élé-ments suivants :

K, Ca, Cs, Ba, Pt, Ni, Sn, Cd, Sr, Li, Mg, Na, Rb, Au, Cu, Zn, La, Ce, Th, Be, Sb, Bi, Ti, Pd, Ir, Os, Ru, Fe, Al.

De même la demanderesse a observé que de façon connue certains dérivés halogénés des hydrocarbures, ajoutés en faibles quantités aux réactifs, augmentent la sélectivité des catalyseurs décrits dans la présente invention. L'emploi du dichloro-1,2 éthane, à une concentration maximale de 1 ppm par rapport au volume total gazeux, s'est révélé par exemple particulièrement intéressant.

Les exemples comparatifs 1 et 2 mettent nettement en évidence la supériorité du catalyseur préparé sur un support possédant une répartition poreuse bimodale, tandis que les exemples 3 à 10 ci-dessous illustrent de façon non limitative la préparation et l'utilisation des catalyseurs selon l'invention.

Les résultats obtenus dans ces exemples sont exprimés en taux de transformation globale de l'éthylène et en sélectivité en oxyde d'éthylène.

- taux de transformation globale de l'éthylène (T.T.G.) :

$$T.T.G. = \frac{\text{Nombre de moles d'éthylène transformées}}{\text{Nombre de moles d'éthylène introduites}} \times 100$$

- sélectivité de la transformation en oxyde d'éthylène
(S.O.E.)

$$S.O.E. = \frac{\text{Nombre de moles d'oxyde d'éthylène formées}}{\text{Nombre de moles d'éthylène transformées}} \times 100$$

EXEMPLE 1 : Essai comparatif

On place dans un ballon à solides, monté sur un évaporateur rotatif, 42,4 g de support SA 5151 fabriqué par la société NORTON et dont les caractéristiques sont rassemblées dans le tableau 1 de la figure 1. On porte la température du bain d'huile de l'évaporateur à 120°C, et on laisse dégazer le support pendant une heure sous une pression partielle de 100 mm de mercure. Dans les mêmes conditions de température et de pression, on introduit ensuite, goutte à goutte, sur le support agité, une solution constituée de 11,7 g d'acétate d'argent dans 220 ml de pyridine. Dans ces conditions, l'évaporation du solvant est instantanée. Après introduction de la totalité de la solution, le support, imprégné et séché, est transféré dans un réacteur tubulaire pour y subir un traitement thermique qui libère le métal. Ce traitement est effectué sous courant d'azote avec une montée de température à 20°C/heure jusqu'à 280°C où l'on reste en palier pendant 18 heures. Le dosage indique une teneur en argent de 13,3 % en poids dans le catalyseur.

On charge 30 ml de ce catalyseur dans un réacteur de laboratoire fonctionnant sous pression, constitué par un tube en acier inoxydable de 355 mm de long, et de 16 mm de diamètre intérieur, chauffé par un bain de nitrates fondus. Les réactifs admis par le bas du réacteur sont préchauffés sur un remplissage en porcelaine de 42 mm de hauteur. Les gaz entrant et sortant du réacteur sont analysés en ligne à l'aide d'un chromatographe à double détection : un détecteur à

- 10 -                    0002977

ionisation de flamme pour l'oxyde d'éthylène, le méthane, le formol, le propylène, le propane, le méthanol et l'acétaldéhyde et un détecteur à conductibilité thermique pour l'oxygène-azote, le dioxyde de carbone, l'éthylène et l'eau. Deux colonnes de diamètre 1/8 de pouce et de longueur 2,5 mètres, montées en série, sont remplies l'une de chromosorb 101, l'autre de porapak Q.

On fait tout d'abord subir au catalyseur un prétraitement d'activation par un mélange éthylène-air 50 % - 50 % à la pression atmosphérique pendant 35 heures entre 168°C et 194°C. On admet ensuite, sous une pression de 20 bars, les réactifs constitués par un mélange de 13 % d'éthylène, 5 % d'oxygène, 82 % d'azote et 35 parties par billion de dichloro-1,2 éthane, à un débit de 9.000 litres normaux/heure par litre de catalyseur. A 198°C, on obtient pour un taux de transformation globale de l'éthylène de 4,1 % une sélectivité en oxyde d'éthylène de 71,2 %.

EXEMPLE 2 : Essai comparatif

On charge dans un ballon à solides, monté sur un évaporateur rotatif 29,5 g de support SA 5205, fabriqué par la société NORTON, et dont les caractéristiques sont rassemblées dans le tableau n° 1 et la figure 1. On opère de la même façon que dans l'exemple 1 et on imprègne le support par une solution de 8 g d'acétate d'argent dans 200 ml de pyridine.

L'analyse, après un traitement thermique effectué d'une façon identique à celui décrit dans l'exemple 1, indique une teneur en argent du catalyseur de 14,9 % en poids. On charge 30 ml de ce catalyseur dans le réacteur fonctionnant sous pression décrit dans l'exemple 1. Après un prétraitement d'activation par un mélange air-éthylène 50 % - 50 %, pendant 36 heures entre 182 et 198°C, effectué à pression atmosphérique, on introduit les réactifs sous 20 bars dans les proportions suivantes : éthylène 13 %, oxygène 5 %, azote 82 %, dichloro-1,2 éthane 35 ppb, débit gazeux 9.000 litres normaux/h/litre de catalyseur. A 199°C, pour un taux de transformation globale de 4,7 %, la sélectivité en oxyde d'éthylène est de 72 %.

EXEMPLE 3

On place dans un ballon à solides, monté sur un évaporateur rotatif 42,5 g de support SA 5239 fabriqué par la Société NORTON et dont les caractéristiques sont rassemblées dans le tableau 1 et la figure 1. On imprègne ce support, d'une manière identique à celle décrite dans l'exemple 1, à l'aide d'une solution de 11,6 g d'acétate d'argent dans 220 ml de pyridine.

La teneur en argent obtenue par dosage, après un traitement thermique effectué dans les mêmes conditions que celles décrites dans l'exemple 1, est de 12,5 % en poids. On charge 30 ml de ce catalyseur dans le réacteur de test décrit dans l'exemple 1 et on fait passer pendant 29 heures à la pression atmosphérique un mélange éthylène-air 50 % - 50 % entre 160 et 176°C. On introduit ensuite les réactifs sous 20 bars, dans les mêmes conditions que celles décrites dans les exemples 1 et 2. A 205°C, pour un T.T.G. de l'éthylène de 5 %, on obtient une sélectivité en oxyde d'éthylène de 75 % nettement supérieure à celles obtenues dans les deux exemples précédents.

EXEMPLE 4

A la suite de l'essai n° 3 on fait passer sur la même charge de catalyseur, sous une pression de 20 bars un courant gazeux de 9.000 litres normaux/heure et par litre de catalyseur, formé d'éthylène, d'oxygène, d'azote et de différentes teneurs en dioxyde de carbone, ainsi que 35 ppb de dichloro-1,2 éthane. On obtient les résultats décrits dans le tableau n° 2.

<u>TABLEAU n° 2</u>

| Gaz entrants | | | | Températ**ture** (°C) | T.T.G. éthylène (%) | S Oxyde d'éthylène (%) |
|---|---|---|---|---|---|---|
| % éthylène | % oxygène | % azote | % $CO_2$ | | | |
| 13 | 5 | 72 | 10 | 221 | 5 | 75 |
| 13 | 5 | 68 | 14 | 221 | 5 | 73 |

<u>EXEMPLE 5</u>

L'objet de cet exemple est d'étudier un catalyseur ayant une forte concentration en argent. On utilise comme support les billes de silice-alumine commercialisées par la société NORTON sous la référence SA 5239 et dont les caractéristiques sont rassemblées dans le tableau n° 1 et la figure n° 1.

On imprègne 36 g de ce support dans des conditions identiques à celles décrites dans l'exemple 1 à l'aide d'une solution de 15 g d'acétate d'argent dans 300 ml de pyridine. Après un traitement thermique identique à celui décrit dans l'exemple 1, le dosage indique une teneur en argent de 20 % en poids dans le catalyseur. On charge 30 ml du catalyseur dans le réacteur de test décrit dans l'exemple 1 et on introduit pendant 28 heures un mélange gazeux d'éthylène-air 50 % - 50 %, à la pression atmosphérique, entre 158°C et 156°C. On fait ensuite passer dans le réacteur un courant gazeux de 9.000 litres normaux/heure/litre catalyseur, sous une pression de 20 bars, formé de 13 % d'éthylène, 5 % d'oxygène, 82 % d'azote et 35 ppb de dichloro-1,2 éthane. Dans ces conditions à 198°C, le taux de transformation global de l'éthylène est de 5 % et la sélectivité en oxyde d'éthylène de 76 %.

EXEMPLE 6

L'imprégnation du support par le composé d'argent peut être menée en milieu purement organique ou en milieu aqueux. Ainsi, sur 29,5 g de support SA 5239 dont les caractéristiques sont décrites dans le tableau n° 1 et la figure 1, on introduit dans des conditions identiques à celles décrites dans l'exemple 1, une solution de 11,8 g d'acétate d'argent dans 220 g d'un mélange eau-pyridine 50 % - 50 %.

Après un traitement thermique identique à celui décrit dans l'exemple 1, la teneur en argent du catalyseur, obtenue par dosage, est de 13 % en poids. On charge 30 ml du catalyseur dans le réacteur décrit dans l'exemple 1 et on y fait passer pendant 32 heures un mélange air-éthylène 50 % - 50 % à la pression atmosphérique entre 157 et 171°C. On introduit ensuite, sous 20 bars, un mélange gazeux de 9.000 litres normaux/heure/litre de catalyseur, formé de 13 % d'éthylène, 5 % d'oxygène, 82 % d'azote et de 35 ppb de dichloro-1,2 éthane. A 204°C, le T.T.G. de l'éthylène est de 5 % et la sélectivité en oxyde d'éthylène de 75 %.

EXEMPLE 7

On imprègne un composé d'argent et un composé de baryum sur 36 g de support SA 5239 dont les caractéristiques sont décrites dans le tableau n° 1 et la figure 1. A l'aide de deux pompes fonctionnant en parallèle, on introduit sur le support placé dans un ballon à solides monté sur un évaporateur rotatif d'une part une solution de 15 g d'acétate d'argent dans 285 ml de pyridine et d'autre part 20,4 ml d'une solution de 1,07 g d'acétate de baryum dans 100 ml d'eau, le tout sous une pression partielle de 100 mm de mercure, le bain d'huile de l'évaporateur étant chauffé à 120°C. Le traitement thermique est identique à celui décrit dans l'exemple 1 et les dosages indiquent que la teneur en argent est de 19 % en poids et celle en baryum de 0,25 % en poids. On charge 30 ml du catalyseur dans le réacteur décrit dans l'exemple 1 et on introduit pendant 45 heures, à la pression atmosphérique, un mélange gazeux air-éthylène 50 % - 50 % entre 150 et 169°C. On fait ensuite passer, sous 20 bars, un mélange gazeux à un débit de 9.000 litres normaux/heure/litre de catalyseur, constitué de 13 % d'éthylène, 5 % d'oxygène, 82 % d'azote et de 35 ppb de dichloro-1,2 éthane. A 180°C, pour un T.T.G. de l'éthylène de 4,5 %, la sélectivité en oxyde d'éthylène est de 75 %.

EXEMPLE 8

On prépare le complexe de l'argent avec le salicylaldéhyde, en ajoutant à une solution de 10,8 g, soit 0,06 mole, de nitrate d'argent dans 50 ml d'eau, une solution de 8 g, soit 0,06 mole de salicylaldéhyde dans 40 ml d'éthanol et, ensuite, goutte à goutte, une solution de 2,6 g de soude dans 80 ml d'eau. A pH = 7,7 il se forme un précipité jaune vert qu'on filtre. On reprend le filtrat dans lequel on continue à ajouter, goutte à goutte la solution de soude jusqu'à ce que le pH atteigne à nouveau 7,7 et qu'apparaisse un nouveau précipité. On recueille celui-ci avec le précédent et on recommence l'opération jusqu'à épuisement du filtrat. L'addition de la soude doit être effectuée avec précautions de façon à éviter le passage brutal à un pH trop basique entrainant la précipitation d'oxyde d'argent. Après plusieurs lavages à l'eau et à l'éthanol du précipité, on recueille après séchage 12,7 g d'un produit vert contenant 46,9 % en poids d'argent, pour une valeur théorique de 47,1 %. Le rendement molaire est de 87 %. Le produit doit être conservé à l'abri de l'air et de la lumière, car il subit une lente décomposition. Suivant le mode opératoire décrit dans l'exemple 1, on prépare un catalyseur à partir d'une solution de 12,7 g du complexe d'argent du salicylaldéhyde ainsi obtenu dans 200 ml de pyridine et de 23,5 g d'un support 5239 fabriqué par la société NORTON et dont les caractéristiques sont rassemblées dans le tableau n° 1 et la figure 1. Le support imprégné et séché est transféré dans un réacteur tubulaire pour décomposer le complexe dans un courant d'azote auquel on ajoute 1,5 % d'hydrogène. Afin de contrôler thermiquement la réaction, ce traitement est effectué avec une montée de température de 20°C/heure jusqu'à un palier de 18 heures à 280°C. Le dosage, effectué après ce traitement, indique une teneur en argent de 20 % en poids dans le catalyseur. On place 30 ml de ce catalyseur dans un réacteur constitué par un tube en acier inoxydable de 600 mm de longueur et de 16 mm de diamètre intérieur. Les réactifs sont admis par le bas et sont préchauffés sur un lit d'anneaux de porcelaine de 200 mm de hauteur, qui supporte également la charge de catalyseur. Le chauffage de l'ensemble est assuré par une circulation d'huile dans une double enveloppe. Les gaz, à l'entrée et à la sortie du réacteur, sont analysés à l'aide d'un dispositif analogue à celui décrit dans l'exemple 1.

On fait passer à travers le lit de catalyseur disposé dans le réacteur un mélange air-éthylène 50 % - 50 %, à la pression atmosphérique, pendant 25 heures, à une température de 190°C. On introduit ensuite dans le réacteur, à la pression atmosphérique, un courant gazeux de 14 l/heure, constitué d'un mélange de 13 % d'éthylène, 5 % d'oxygène, 82 % d'azo-

te et de 640 ppb de dichloro-1,2 éthane. Après 60 heures de marche, on obtient les résultats donnés dans le tableau n° 3 :

TABLEAU n° 3

| T°C du catalyseur | % T.T.G. | % S.O.E. |
|---|---|---|
| 180 | 5 | 76 |
| 194 | 10 | 73 |

### EXEMPLE 9

La charge de catalyseur de l'exemple 8 est transférée dans le réacteur fonctionnant sous 20 bars décrit dans l'exemple 1. On introduit un courant gazeux de débit horaire spécifique de 9.000 litres normaux par litre de catalyseur, formé de 13 % d'éthylène, 5 % d'oxygène, 82 % d'azote et 35 ppb de dichloro-1,2 éthane. Après 27 heures de marche, on obtient à 206°C une conversion de l'éthylène de 5 % avec une sélectivité en oxyde d'éthylène de 76 %.

### EXEMPLE 10

On prépare le sel d'argent de l'acide salicylique en ajoutant une solution de 17 g, soit 0,10 mole de nitrate d'argent dans 30 ml d'eau à une solution de 13,8 g, soit 0,10 mole d'acide salicylique dans 200 ml d'éthanol. On coule ensuite lentement dans le mélange 20 ml d'une solution d'ammoniaque 5N. Le précipité blanc qui se forme immédiatement est lavé à l'eau, filtré et séché. On recueille 23 g de salicylate d'argent correspondant à un rendement molaire de 94 %. La teneur en argent correspond à la théorie : 44 %.

Suivant le mode opératoire décrit dans l'exemple 1, on prépare un catalyseur à partir d'une solution pyridinique de 18 g du salicylate d'argent préparé ci-dessus et 32 g de support 5239 fabriqué par la société NORTON et dont les caractéristiques sont rassemblées dans le tableau n° 1 et la figure 1. Après un traitement thermique identique à celui décrit dans l'exemple 8, le dosage indique une teneur en argent de 11,2 % en poids dans le catalyseur. On place 30 ml de ce catalyseur dans le réacteur décrit dans l'exemple 8 et on fait passer dans celui-ci à la pression atmosphérique pendant 14 heures, un mélange air-éthylène 50 % - 50 % à 215 - 250°C. On introduit ensuite à la pression atmosphérique, 14 litres/heure d'un gaz contenant 14 % d'éthylène, 5 % d'oxygène, 81 % d'azote et 540 ppb de dichloro-1,2 éthane. Après 28 heures de marche on obtient les résultats donnés dans le tableau n° 4.

TABLEAU n° 4

| T°C du catalyseur | % T.T.G. | % S.O.E. |
|---|---|---|
| 206 | 5 | 77 |
| 227 | 10 | 74 |

- 1 -

Revendications de brevet

1. Catalyseurs à base d'argent pour la synthèse en phase vapeur d'oxyde d'éthylène par réaction d'oxygène ou de mélanges gazeux en contenant sur de l'éthylène, caractérisés par l'emploi comme support d'un matériau poreux présentant une surface spécifique inférieure à 10 $m^2$/g, un volume total de porosité compris entre 0,1 et 0,6 $cm^3$/g et une répartition bimodale de la porosité où les plus petits pores représentant 35 à 65 % du volume des pores ont un diamètre moyen compris entre 1 et 5 µ, les gros pores ayant un diamètre moyen compris entre 60 et 200 µ.

2. Catalyseurs selon la revendication 1 où les plus petits pores de diamètre moyen compris entre 1 et 5 µ représentent de 45 à 55 % du volume des pores.

3. Catalyseurs selon l'une des revendications 1 ou 2 caractérisés par le fait que le support est essentiellement formé d'alumine ou de silice-alumine.

4. Catalyseurs selon l'une des revendications 1 à 3 caractérisés par le fait qu'ils contiennent 5 à 25 % en poids d'argent et sont préparés par imprégnation du support par une solution anhydre ou aqueuse d'un composé d'argent suivi d'un traitement thermique libérant le métal.

0002977

5. Catalyseurs selon l'une des revendications 1 à 4 dans lesquels on introduit de faibles quantités de baryum sous forme d'oxyde ou de sel.

6. Procédé de préparation d'oxyde d'éthylène par réaction d'oxygène, ou de mélange gazeux contenant de l'oxygène, sur de l'éthylène caractérisé par l'emploi d'un catalyseur selon l'une des revendications 1 à 5.

0002977

PL. unique

FIG.1

))) Office européen
des brevets

**RAPPORT DE RECHERCHE EUROPEENNE**

0002977

Numéro de la demande

EP 78 40 0234

| Catégorie | Citation du document avec indication, en cas de besoin, des parties pertinentes | Revendica-tion concernee | CLASSEMENT DE LA DEMANDE (Int. Cl.²) |
|---|---|---|---|
| | **DOCUMENTS CONSIDERES COMME PERTINENTS** | | |
| X | <u>DE - A - 2 723 918</u> (ICI)<br><br>* Pages 1-3; revendications * | 1-6 | C 07 D 301/10<br>B 01 J 23/50<br>35/10<br>C 07 D 303/04 |

**DOMAINES TECHNIQUES RECHERCHES (Int. Cl.²)**

C 07 D 301/10
B 01 J 23/50
23/66
35/10
C 07 D 303/04

**CATEGORIE DES DOCUMENTS CITES**

X: particulièrement pertinent

A: arrière-plan technologique

O: divulgation non-écrite

P: document intercalaire

T: théorie ou principe à la base de l'invention

E: demande faisant interférence

D: document cité dans la demande

L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 23-03-1979 | ALLARD |

OEB Form 1503.1 06.78